(19) Europäisches Patentamt
European Patent Office
Office européen des brevets

(11) Publication number: 0 323 737 A1

(12) # EUROPEAN PATENT APPLICATION

(21) Application number: 88312197.2

(22) Date of filing: 22.12.88

(51) Int. Cl.4: C07D 249/12 , C07C 159/00 , A61K 31/41

Claims for the following Contracting States: ES + GR

(30) Priority: 31.12.87 US 140074

(43) Date of publication of application: 12.07.89 Bulletin 89/28

(84) Designated Contracting States: AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Kruse, Lawrence Ivan
33 Firs Walk
Tewin Hertfordshire AL7 0NY(GB)
Inventor: Finkelstein, Joseph Alan
7549 Brentwood Road
Philadelphia Pennsylvania 19151(US)

(74) Representative: Waters, David Martin, Dr. et al
Smith Kline & French Laboratories Ltd.
Patent Department Mundells
Welwyn Garden City Hertfordshire AL7 1EY(GB)

(54) **4-Aralkyl-5-substituted-1,2,4-triazole-5-thiols.**

(57) Compounds of formula (I)

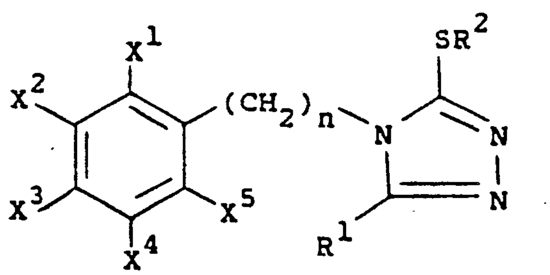

(I)

and pharmaceutically acceptable salts thereof are described in which, n is 0 to 5; $X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, OH, $CH_2OH$, $CO_2H$, or $CO_2C_pH_{2p+1}$ wherein p is 1 to 4; $R^1$ is phenyl substituted by $X^1$ to $X^5$, $C_{1-4}$alkyl, $C_{3-6}$cycloalkyl, or an aryl$C_{1-4}$alkyl group substituted by $X^1$ to $X^5$; $R^2$ is hydrogen, $C_{1-4}$alkyl or $(CH_2)_m$-$CO_2R^3$; m is 0 to 5; and $R^3$ is H or $C_{1-4}$alkyl.

These compounds are dopamine-β-hydroxylase inhibitors. Pharmaceutical compositions are described as are methods of use. Processes for the preparation of these compounds are described.

EP 0 323 737 A1

# 4-ARALKYL-5-SUBSTITUTED-1,2,4-TRIAZOLE-5-THIOLS

The present invention relates to novel substituted 4-aralkyl-5-substituted-1,2,4-triazole-3-thiols, processes for their preparation, intermediates useful in their preparation, pharmaceutical compositions containing them and their use in therapy in particular as DBH inhibitors.

Compounds that inhibit DBH activity are well known in the art and include:

(a) 5-alkylpicolinic acids [See, Suda et al., Chem. Pharm. Bull. 17, 2377 (1969); Umezawa et al., Biochem. Pharmacol. 19, 35 (1969); Hidaka et al., Mol. Pharmacol. 9, 1972 (1973); Miyano et al., Chem. Pharm. Bull. 26, 2328 (1978); Miyano et al., Heterocycles 14, 755 (1980); Claxton et al., Eur. J. Pharmacol. 37, 179 (1976)];

(b) BRL 8242 [See, Claxton et al., Eur. J. Pharmacol. 37, 179 (1976)];

(c) 1-alkylimidazole-2-thiols [See, Hanlon et al., Life Sci. 12, 417 (1973); Fuller et al., Adv. Enzyme Regul. 15 267 (1976)];

(d) substituted thioureas [See, Johnson et al , J. Pharmacol. Exp. Ther. 168, 229 (1969)]; and

(e) benzyloxamine and benzylhydrazine [See, Creveling et al., Biochim. Biophys. Acta 64, 125 (1962); Creveling et al., Biochim, Biophys. Acta 8, 215 (1962); Van De Schoot et al., J. Pharmacol. Exp. Ther. 141, 74 (1963); Bloom, Ann N.Y. Acad. Sci. 107, 878 (1963)].

(f) fusaric acid derivatives and analogues [See, Runti et al., Il Farmaco Ed. Sci. 36, 260 (1980)] for example phenylpicolinic acid, 5-(4-chlorobutyl) picolinic acid, substituted amides of fusaric acid and acids and amides of 5-butydropicolinic acid, 5-aminopicolinic acid, 5-hydrazinopicolinic acid, and derivatives thereof.

(g) Hidaka et al., Molecular Pharmacology, 9, 172-177 (1972) 5-(3,4-dibromobutyl)picolinic acid and 5-(dimethyldithiocarbamoyl)methylpicolinic acid.

(h) Bupicomide, 5-(n-butyl)picolinamide, is reported by Ehrreich et al., "New Antihypertensive Drugs", Spectrum Publications, 1976, pg. 409-432,

(i) In United States Patent No. 4,532,331 a series of 1-phenyl and 1-phenylalkylimidazole compounds having a mercapto or alkylthio group in the 2-position are disclosed.

(j) United States Patent No. 4,487,761 describes several methylpyridine derivatives isolated from the fermentation broth of a strain of Streptoverticillium.

(k) Friedman et al., Psychosomatic Med. 40, 107 (1978), report that patients treated with alpha-methyl- DOPA, guanethidine and reserpine but not propranolol and diuretics, have lowered DBH levels, although the significance of the observation is uncertain.

(l) In United States Patent No. 3,448,423 are disclosed compounds having the formula:

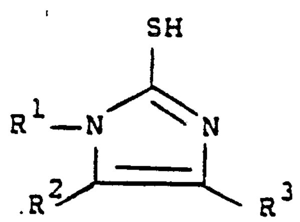

in which $R^2$ and $R^3$ can be H, and $R^1$ can be substituted phenyl. The compounds are said to have analgesic, anti- inflammatory and antipyretic properties. Gerbert et al., US Patent 3,915,980, disclose such compounds wherein $R^1$ can be phenyl or phen($C_{1-3}$)alkyl, as intermediates to imidazolyl-2-thioalkanoic acid esters.

(m) Iverson, Acta Chem. Scan. 21, 279 (1967) reports compounds having the formula :

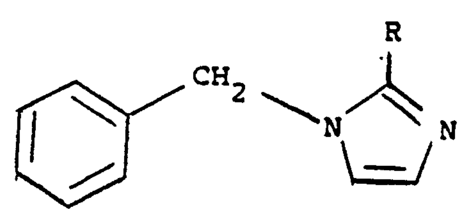

wherein R can be $-CO_2H$ or $-CH_2NHC_6H_5$, but does not report pharmaceutical uses for the compounds.

In addition to the foregoing, a number of compounds which are structurally related to those of the present invention are also known, however, no DBH activity has been attributed to them;

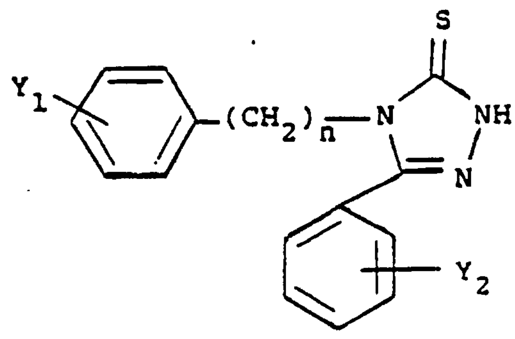

For example, compounds of the above noted structure in which n is O, $Y^1$ is hydrogen and $Y^2$ is one or more substituents selected from hydrogen, halogen, hydroxy or alkoxy are disclosed in Bany, T. et al. Ann Univ. Mariae Curie-Sklodowska Sect AA, pp. 29-30, 163-169, 1976; Tandon, M. et al. Indian J.Chem. 20B-(11):1017-1018, 1981; Shah, M.H. et al. J. Pharm. Sci. 58(11):1398-1401, 1969; Jaiswal, R.K. et al. J. Heterocycl. Chem. 16(3):561-565, 1979; Mazzone, G. et al. Farmaco Ed.Sci. 36(3):181-196, 1981; compounds in which n is O, and $Y^1$ is a 2-methyl or 2-methoxy substituent and $Y^2$ is hydrogen, alkyl, alkoxy, hydroxy, nitro or halogen are disclosed in Rao, V.R. and Srinivasan, V.R. Symp. Syn. Heterocycl. Compounds Physiol Interest, pp. 137-144, 1964; Shukla, J.S. et al J.Prakt. Chem. 311(3):523 526, 1969; Nath, T.G. et al. Indian J. Chem. 15B(4): 341- 346, 1977; compounds in which n is O, $Y^1$ is a 3-methyl or 3-halo group and $Y^2$ is selected from hydrogen, alkyl, alkoxy, halogen or hydroxy are disclosed in Hazzaa, A.A.B and Shafik, R.M. Egypt J. Pharm. Sci. 19(1-4):201-206, 1978; Nath, T.G. et al. Indian J. Chem. 15B-(4): 341-346, 1977; Shukla, J.S. et al. J.Prakt.Chem. 311(3): 523-526, 1969; Srivastava, U. et al. Bokin Bobai 7(9):T414-T417, 1979; compounds in which n is O, $Y^1$ is a 4-methyl, 4-alkoxy or 4-halo group and $Y^2$ is hydrogen, alkoxy, hydroxy, halogen or nitro are disclosed in Shukla, J.S. et al. J.Prakt.Chem. 311(3): 523-526, 1969; Tandon, M. et al. Indian J.Chem. 20B(11):1017-1018, 1981; Bhat, A. K. et al. Indian J.Chem. 5-(9):397-401, 1967; Bany, T. et al. Ann Univ. Mariae Curie-Sklodowska Sect AA, pp. 29-30, 163-169, 1976; Joshni, K.C. and Mehta, D.S. J. Indian Chem. Soc. 51(6):613-615, 1974; compounds in which n is O, $Y^1$ is 3,4-methyl or 2,4-methyl and $Y^2$ is 3,4,5-methoxy group are disclosed in Jaiswal, R.K. et al. J.Heterocycl.Chem. 16(3): 561-565, 1979; compounds in which n is O, $Y^1$ is a 3,4-chloro and $Y^2$ is 2-hydroxy-4-bromo or 4-fluoro are disclosed in Bhat, A.K. et al. Indian J.Chem. 5(9):397-401, 1967; Joshni, K.C. and Mehta, D.S. J.Indian Chem. Soc. 51(6):613-615, 1974; and compounds in which n is 1, $Y^1$ ishydrogen and $Y^2$ is hydrogen, methyl, methoxy, halogen or $NO_2$ are disclosed in Vakula, T.R. et al. Indian J. Chem. 7(6):577-580, 1969. The compounds disclosed in the fore- going references are disclosed as synthetic intermediates or as antimicrobial agents.

Non-specific, often toxic effects of known DBH inhibitors have obviated clinical use of these compounds. Fusaric acid, for example, is hepatotoxic. See, for example, Teresawa et al., Japan Cir. J. 35, 339 (1971) and references cited therein.

Therefore there is a continuing need for novel compounds that possess DBH inhibitory activity.

Accordingly the present invention provides compounds of structure (I):

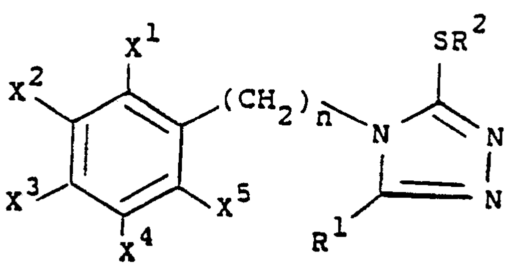

(I)

in which,

n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$ $SO_2CH_3$, $SO_2CH_2F$, $SO_2CHF_2$, $SO_2CF_3$, $CF_3$, CHO, OH, $CH_2OH$, $CO_2H$, or $CO_2C_pH_{2p+1}$ wherein p is 1 to 4;
$R^1$ is phenyl substituted by $X^1$to $X^5$, $C_{1-4}$alkyl, $C_{3-6}$cycloalkyl, or an aryl $C_{1-4}$ alkyl group substituted by $X^1$ to $X^5$;
$R^2$ is hydrogen, $C_{1-4}$alkyl or $(CH_2)_m$-$CO_2R^3$;
m is 0 to 5; and
$R^3$ is H or $C_{1-4}$alkyl;
or pharmaceutically acceptable salts thereof provided that

(i) when n is O, $R^2$ is hydrogen and $X^1$ to $X^5$ are hydrogen, $R^1$ is other than phenyl or phenyl substituted by OH, $C_{1-6}$alkoxy, halogen;

(ii) when n is O, $R^2$ is hydrogen, $X^1$ is $C_{1-6}$alkyl or $C_{1-6}$alkoxy and $X^2$ to $X^5$ are hydrogen, $R^1$ is other than phenyl or phenyl substituted by $C_{1-6}$alkyl, $C_{1-6}$alkoxy hydroxy, nitro or halogen;

(iii) when n is O, $R^2$ is hydrogen, $X^2$ is $C_{1-6}$alkyl or halogen and $X^1$ and $X^3$ to $X^5$ are hydrogen, $R^1$ is other than phenyl or phenyl substituted by $C_{1-6}$alkyl, $C_{1-6}$alkoxy, hydroxy or halogen;

(iv) when n is O, $R^2$ is hydrogen, $X^1$, $X^2$ and $X^4$, $X^5$ are hydrogen and $X^3$ is $C_{1-6}$alkyl, halogen or $C_{1-6}$alkoxy, $R^1$ is other than phenyl or phenyl substituted by $C_{1-6}$alkoxy, hydroxy, halogen or nitro;

(v) when n is O, $R^2$ is hydrogen, $X^4$ and $X^5$ are hydrogen, one of $X^1$ and $X^2$ is $C_{1-6}$alkyl and the other is hydrogen and $X^3$ is $C_{1-6}$alkyl, $R^1$ is other than a phenyl group substituted by three $C_{1-6}$alkoxy groups;

(vi) when n is O, $R^2$ is hydrogen, $X^1$, $X^4$ and $X^5$ are hydrogen and $X^2$ and $X^3$ are halogen, $R^1$ is other than a phenyl group substituted by hydroxy or halogen; and

(vii) when n is 1, $R^2$ is hydrogen and $X^1$ to $X^5$ are all hydrogen, $R^1$ is other than phenyl, or a phenyl group monosubstituted by methyl, methoxy, chloro or nitro, or a phenyl group disubstituted by chloro.

As used herein "accessible combination" means any combination of the substituents that is available by chemical synthesis and is stable

It will be appreciated that when $R^2$ is hydrogen Structure (I) covers the tautomeric forms thereof that is compounds of structure (Ia)

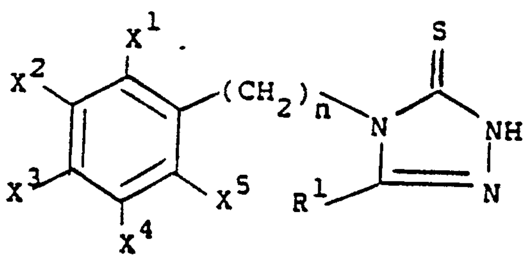

(Ia)

Suitably n is 0 to 5, preferably 0 or 1, most preferably 1.

Suitably $X^1$ to $X^5$ are all hydrogen. More suitably at least one of $X^1$ to $X^5$ is halogen and the others are hydrogen. Preferably, $X^2$ or $X^4$ is halogen or $X^4$ and $X^2$ are halogen and $X^1$, $X^3$ and $X^5$ are all hydrogen. More preferably $X^2$ and $X^4$ are halogen, $X^1$ and $X^5$ are hydrogen and $X^3$ is $C_{1-6}$alkoxy. Most preferably $X^2$ and $X^4$ are fluorine; $X^1$ and $X^5$ are hydrogen and $X^3$ is methoxy.

Suitably $R^1$ is phenyl. Preferably $R^1$ is a substituted phenyl group. Most preferably $R^1$ is a phenyl group substituted by a single substituent, in particular a $C_{1-6}$ alkyl group, such as t-butyl in the 4-position of the ring.

It is to be noted that $C_{1-6}$alkyl groups either alone or as part of another group (e.g. aryl $C_{1-6}$alkyl) can be straight or branched.

Particular compounds of this invention include:
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole,
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,

3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2,4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,
3-mercapto-4 [3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n-propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,
3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(3,4,5-trimethoxyphenyl)-1,2,4-triazole,
3-mercapto-4-benzyl-5-(4-bromophenyl)-1,2,4-triazole,
and
3-mercapto-4-benzyl-5-(3-bromophenyl)-1,2,4-triazole.

A further aspect of the invention provides a process for preparation of compounds of structure (I) and pharmaceutically acceptable salts thereof which comprises cyclization of a compound of structure (II)

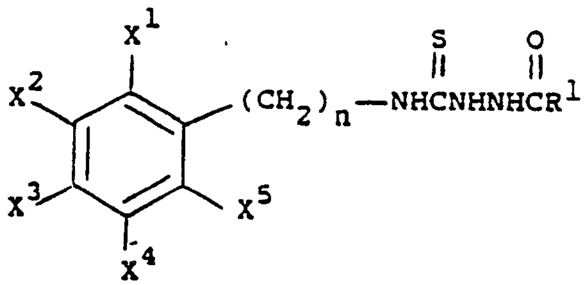

(II)

in which $X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$ $SO_2CH_2F$, $SO_2CHF_2$, $SO_2CF_3$, $CF_3$, CHO, $CH_2OC_{1-6}$alkyl, or $CO_2C_{1-6}$alkyl; and n and $R'$ are as described for structure (I); and optionally thereafter converting a group $X^1$ to $X^5$ into a OH, $CH_2OH$ or $CO_2H$ group, converting a compound of structure (I) in which $R^2$ is hydrogen to one in which $R^2$ is $C_{1-4}$alkyl or $(CH_2)_mCO_2R^3$ in which m and $R^3$ are as hereinbefore defined and optionally forming a pharmaceutically acceptable salt.

The cyclization is carried out in a suitable solvent in the presence of a base. In particular the reaction is carried out in ethanol in the presence of sodium ethoxide as the base.

Compounds of structure (II) are prepared by reaction of a compound of structure (III) and a compound of structure (IV)

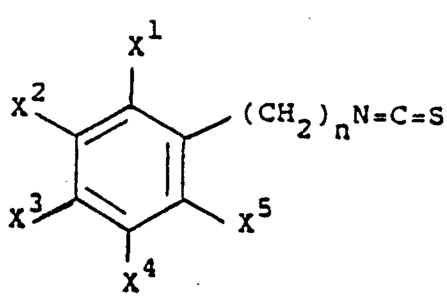

(III)

$R^1CONHNH_2$ (IV)

in which $X^1$ to $X^5$, n and $R'$ are as described for structure (II).

The reaction is carried out in an inert solvent at elevated temperature. Suitable solvents include for example $C_{1-6}$alkanols such as methanol or ethanol, tetrahydrofuran and ethyl acetate; preferably ethanol.

Compounds of structures (III) and (IV) are prepared by methods analogous to those known in the art or are available commercially, for example, compounds of structure (III) are prepared from compounds of structure (V)

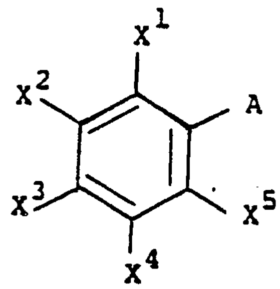

(V)

in which $X^1$ to $X^5$ are as described for structure (II) and A is CN, by reduction with, for example, hydrogen and ammonia in the presence of a Raney alloy to form a compound of structure (V) in which A is $CH_2NH_2$; followed by reaction with, for example, thiophosgene in the presence of a base to form the desired compounds of structure (III).

It is to be noted that as an alternative to preparation and isolation of intermediate (II) by reaction of a compound of structures (III) and (IV) as hereinabove described, the compounds of structures (III) and (IV) may be reacted together and the product cyclized in a single step to form the desired compounds of structure (I) Suitable conditions include for example, heating the compounds (III) and (IV) optionally in the presence of a solvent, at elevated temperature for a suitable time, followed by addition of a suitable base, for example, sodium ethoxide in ethanol to effect the cyclization.

Compounds of the invention in which $R^2$ is $C_{1-4}$alkyl are prepared by alkylating the corresponding compound of structure (I) where $R^2$ is hydrogen with an alkyl halide in the presence of a base, for example, methyl iodide in methanol in the presence of potassium carbonate, by procedures known to those skilled in the art. Other alkyl reagents such as methyl bromide or dimethyl sulphate, in appropriate solvents in the presence of a base, can be substituted for methyl iodide. Further, the compounds of structure (I) in which $R^2$ is an alkyl group other than methyl are prepared by substituting an alkyl halide such as butyl iodide, for the methyl halide to yield the desired substituted 4-aralkyl-5-substituted-1,2,4-triazole-3-thiols of the invention.

Compounds of structure (I) in which $R^3$ is $C_{1-4}$alkyl are prepared by reacting the corresponding compound of structure (I) where $R^2$ is hydrogen with a haloalkanoate ester in the presence of base by procedures known to those skilled in the art. Compounds of structure (I) in which $R^3$ is hydrogen are prepared by mild acid or base hydrolysis of structure (I) compounds in which $R^3$ is$C_{1-4}$alkyl by procedures known to those skilled in the art.

Pharmaceutically acceptable acid addition salts of compounds of the invention are formed with appropriate strong or moderately strong organic or inorganic acids by methods known in the art. For example, the base is reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent.

Exemplary of the salts which are included in this invention include maleate, fumarate, lactate, oxalate, methanesulfonate, ethanesulfonate, benzenesulfonate, tartrate, citrate, hydrochloride, hydrobromide, sulfate, phosphate, quinate, and nitrate salts.

Pharmaceutically acceptable base addition salts of compounds of the invention containing an acidic group (R is $(CH_2)_m$-$CO_2R^3$ and $R^3$ is H) are prepared by known methods from organic and inorganic bases including nontoxic alkali metal and alkaline earth bases, for example, calcium, sodium, and potassium hydroxide; ammonium hydroxide, and nontoxic organic bases such as trimethylamine, triethylamine, propylamine, butylamine, piperazine, and (trihydroxymethyl)methylamine.

The present invention also provides a compound of structure (Ib)

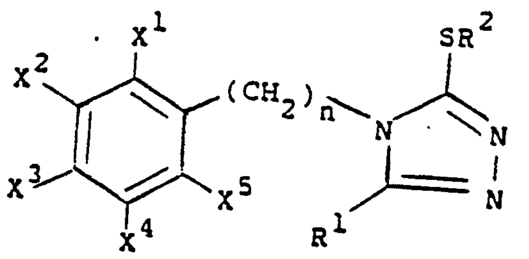

(Ib)

in which,
n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CH_3$, $CF_3$, CHO, OH, $CH_2OH$, $CO_2H$, or $CO_2C_pH_{2p+1}$ wherein p is 1 to 4;
$R^1$ is phenyl substituted by the groups $X^1$to $X^5$, $C_{1-4}$alkyl, $C_{3-6}$cycloalkyl, or an aryl$C_{1-4}$alkyl group substituted by $X^1$ to $X^5$ as described above;
$R^2$ is hydrogen, $C_{1-4}$alkyl or $(CH_2)_m$-$CO_2R^3$;
m is 0 to 5; and
$R^3$ is H or $C_{1-4}$alkyl;
or a pharmaceutically acceptable salt thereof for use as a medicament.

Particular compounds of structure (Ib) include:
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole,
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-benzyl-5-phenyl-1,2,4-triazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2,4-triazole,
3-mercapto-4-[3-(3,5-difluorophenyl)propyl]-5-(4-t-butylphenyl)-1,2 4-triazole,
3-mercapto-4-[3-(3,5-difluoro-4-methoxyphenyl)propyl]-5-(4-t-butylphenyl)-1,2,4-triazole,
3-mercapto-4-[3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n-propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,
3-mercapto-4-benzyl-5-cyclohexyl-1,2,4-triazole,
3 mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(4-methoxyphenyl)-1,2,4-triazole,
3-mercapto-4-benzyl-5-(3,4,5-trimethoxyphenyl)-1,2,4-triazole,
3-mercapto 4-benzyl-5-(4-chlorophenyl-1,2,4-triazole,
3-mercapto-4-benzyl-5-(4-bromophenyl)-1,2,4-triazole,
or
3-mercapto-4-benzyl-5-(3-bromophenyl)-1,2,4-triazole,
or a pharmaceutically acceptable salt thereof.

In a further aspect the present invention provides the use of compounds of structure (Ib) in the manufacture of a medicament for inhibiting dopamine-β-hydroxylase activity.

Because the compounds of structure (I) inhibit DBH activity, they have therapeutic value as diuretic, natriuretic, cardiotonic, antihypertensive, and vasodilatoragents, as well as antiulcerogenic and anti-Parkinson agents. Listed in Table I are the compounds of the invention that were tested for in vitro DBH inhibition by a standard procedure for assaying conversion of tyramine to octopamine in the presence of DBH. J.J.

Pisano, et al., Biochim. Biophys. Acta, 43, 566-568 (1960). Octopamine was assayed following sodium periodate oxidation to p-hydroxybenzaldehyde by measuring spectrophotometric absorbance at 330 nm. In Table I, inhibition is given in molar concentration of compound at which DBH activity was halved ($IC_{50}$). By this test, fusaric acid had an $IC_{50}$ of $8X10^{-7}$ molar.

Table I

| Example | DBH $IC_{50}$ (M) |
|---|---|
| 2 | $1.2 \times 10^{-5}$ |
| 4 | $1.1 \times 10^{-4}$ |
| 8 | $7.4 \times 10^{-6}$ |
| 12 | $5.7 \times 10^{-6}$ |
| 13 | $8.3 \times 10^{-6}$ |
| 15 | $6.4 \times 10^{-7}$ |
| 17 | $5.0 \times 10^{-7}$ |
| 18 | $7.7 \times 10^{-7}$ |
| 19 | $4.6 \times 10^{-7}$ |
| 21 | $3.2 \times 10^{-7}$ |
| 23 | $3.8 \times 10^{-7}$ |
| 25 | $4.3 \times 10^{-7}$ |
| 26 | $7.8 \times 10^{-7}$ |
| 28 | $3.0 \times 10^{-7}$ |
| 30 | $3.0 \times 10^{-7}$ |
| 31 | $1.25 \times 10^{-6}$ |
| 32 | $2.1 \times 10^{-5}$ |
| 33 | $4.6 \times 10^{-5}$ |
| 34 | $9.0 \times 10^{-6}$ |
| 35 | $1.6 \times 10^{-6}$ |
| 36 | $5.5 \times 10^{-7}$ |
| 37 | $1.5 \times 10^{-5}$ |
| 40 | $1.1 \times 10^{-4}$ |
| 41 | $2.3 \times 10^{-5}$ |
| 43 | $5.9 \times 10^{-6}$ |
| 44 | $1.1 \times 10^{-6}$ |
| 45 | $2.1 \times 10^{-5}$ |
| 46 | $2.8 \times 10^{-6}$ |
| 47 | $1.8 \times 10^{-6}$ |
| 48 | $1.6 \times 10^{-6}$ |

Further, spontaneously hypertensive rats were treated with a suspension or solution of 3-mercapto-4-benzyl-5-n- heptyl-1,2,4-triazole at a dose of 50 mg/kg orally, and mean arterial blood pressure was monitored for 260 minutes using indwelling cannulae in the tail arteries. When compared to vehicle-treated controls, animals treated with the compounds of the invention exhibited significant blood pressure reductions within approximately 30 minutes after treatment. Maximal blood pressure reduction was approximately 10 to 35 mm Hg.

The present invention also thus provides compounds of structure (Ib) for use in lowering blood pressure in a mammal, including a human.

The compounds of structure (Ib) usually are administered in a standard pharmaceutical composition. The present invention therefore provides in a further aspect pharmaceutical compositions comprising a compound of structure (Ib) or a pharmaceutically salt thereof and a pharmaceutically acceptable carrier. Such compositions include those suitable for administration via an appropriate route known to those skilled in the art for example, orally, parenterally, transdermally, rectally, via inhalation or via buccal administration.

The compounds of structure (1b) and their pharmaceutically acceptable salts which are active when given orally can be formulated as tablets, capsules, lozenges and liquids, for example, syrups, suspensions or emulsions.

A liquid formulation generally will consist of a suspension or solution of the compound or pharmaceutically acceptable salt in a suitable liquid carrier(s) for example, ethanol, glycerine, sorbitol, non-aqueous

solvent, for example polyethylene glycol, oils, or water with a suspending agent, preservative surfactant, wetting agent, flavouring or colouring agent.

Alternatively, a liquid formulation is prepared from a reconstitutable powder. For example a powder containing active compound, suspending agent, sucrose and a sweetener is reconstituted with water to form a suspension; and a syrup is prepared from a powder containing active ingredient, sucrose and a sweetener

A composition in the form of a tablet is prepared using any suitable pharmaceutical carrier(s) routinely used for preparing solid formulations. Examples of such carriers include magnesium stearate, starch, lactose, sucrose, cellulose and binders, for example, polyvinylpyrrolidone. The tablet optionally is provided with a color film coating, or color included as part of the carrier(s). In addition, acting compound can be formulated in a controlled release dosage form such as a tablet comprising a hydrophilic or hydrophobic matrix.

A composition in the form of a capsule is prepared using routine encapsulation procedures. For example, pellets containing active ingredient are prepared using standard carriers and then filled into a hard gelatin capsule; alternatively, a dispersion or suspension is prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilized and then reconstituted with a suitable solvent just prior to administration.

A typical suppository formulation comprises a compound of structure (Ib) or a pharmaceutically acceptable salt thereof which is active when administered in this way, with a binding and/or lubricating agent such as polymeric glycols, gelatins or cocoa butter or other low melting vegetable or synthetic waxes or fats.

Compounds of structure (Ib) and their pharmaceutically acceptable addition salts which are active on topical administration can be formulated as transdermal compositions. Such compositions include, for example, a backing, active compound reservoir, a control membrane, liner and contact adhesive.

Typical compositions for inhalation are in the form of a solution, suspension or emulsion that may be administered in the form of an aerosol using a conventional propellant such a dichlorodifluoromethane or trichlorofluoromethane.

Preferably the composition is in an appropriate unit dosage form. Each dosage unit for oral administration contains preferably from 1 to 250 mg (and for parenteral administration contains preferably from 0.1 to 25 mg) of a compound of the structure (Ib) or a pharmaceutically acceptable salt thereof calculated as the free acid or base.

The daily dosage regimen for an adult patient may be, for example, an oral dose of between 1 mg and 1000 mg, preferably between 1 mg and 250 mg, or an intravenous subcutaneous, or intramuscular dose of between 0.1 mg and 100 mg, preferably between 0.1 mg and 25 mg, of the compound of the structure (Ib) or a pharmaceutically acceptable salt thereof calculated as the free base, the compound being administered 1 to 4 or more times per day. Suitably the compounds are administered for a period of continuous therapy, for example for a week or more. In addition, the compounds of this invention may be co-administered with other pharmaceutically active compounds, for example in combination, concurrently or sequentially.

The following Examples illustrate the invention. Temperatures are recorded in degrees centigrade.

## Example 1

1-Benzoyl-4-benzylthiosemicarbazide

Benzyl isothiocyanate (6.63 ml, 0.05 mole) was added to a suspension of benzoylhydrazine (6.81 g, 0.05 mole) in ethanol (70 ml) and the mixture was heated at 50-60 °C for 30 minutes. The mixture was diluted with ethanol (30 ml), cooled in ice and the solid was filtered. The solid wasthen triturated with hot ethanol (200 ml), cooled in ice and the product was filtered to give a solid melting at 188-190 °C (10.2 g, 71%).

## Example 2

3-Mercapto-4-benzyl-5-phenyl-1,2,4-triazole

1-Benzoyl-4-benzylthiosemicarbazide (5. 0 g, 0.0175 mole) was added to a solution of sodium ethoxide [from sodium (0.81 g, 0.035 mole) in ethanol (70 ml)] and the solution was heated at reflux for 16 hours. The solvent was removed under vacuum and the residue was dissolved in water (100 ml), cooled in ice and acidified with 10% hydrochloric acid. The product was filtered, recrystallized from ethanol and dried at 50°C to give a solid melting at 184-185°C (3.82 g, 82%).

Example 3

1-Benzoyl-4-methylthiosemicarbazide

Following the method of Example 1, methylisothio- cyanate (3.66 g, 0.05 mole) and benzoylhydrazine (6.81 g, 350.05 mole) gave a solid melting at 188.5-190.5°C (9.71 g, 92%).

Example 4

3-Mercapto-4-methyl-5-phenyl-1,2,4-triazole

Following the method of Example 2, 1-benzoyl-4-methylthiosemicarbazide (9.0 g, 0.043 mole) and sodium ethoxide [from sodium (1.98 g, 0.086 mole) in ethanol (200 ml)] gave the product which was recrystallized from ethanol with melting point 165-166°C (7.03 g, 85%).

Example 5

3,5-Difluorobenzylamine

A slurry of Raney nickel in methanol was added to a solution of 3,5-difluorobenzonitrile (6.5 g, 0.0467 mole) in methanol (100 ml) saturated with ammonia and the mixture was hydrogenated for 2.25 hours at 50 lbs. pressure. The solution was decanted from the catalyst and the catalyst washed four times with methanol and decanted. The combined decanted solvent was evaporated and the residue dissolved in ethyl acetate and extracted twice with 1N hydrochloric acid (50 ml). The acid solution was made basic with 10% sodium hydroxide and extracted with three portions of ethyl acetate. The ethyl acetate was washed with water, brine, dried over sodium sulfate and the solvent removed to give the product as an oil (6.2 g, 93%).

Example 6

3,5-Difluorobenzylisothiocyanate

A solution of 3,5-difluorobenzylamine (6.2 g, 0.043 mole) and triethylamine (13.3 ml, 0.0953 mole) in dry tetrahydrofuran (35 ml) was added dropwise to thiophosgene (3.6 ml, 0.048 mole) in dry tetrahydrofuran (30 ml) with ice cooling. After stirring at 25°C for 2 hours the mixture was diluted with ether and filtered. The filtrate was treated twice with activated carbon, filtered and the solvent was removed at reduced pressure.

The residue was distilled under vacuum to give the product as an oil (4.58 g, 57%).

Example 7

1-Benzoyl-4-(3,5-difluorobenzyl)thiosemicarbazide

Following the method of Example 1, 3,5-difluoro- benzylisothiocyanate (4.50 g, 0.0243 mole) and benzoylhydrazine (3.31 g, 0.0243 mole) gave a solid melting at 182-190 °C (5.80 g, 74%).

Example 8

3-Mercapto-4-(3,5-difluorobenzyl)-5-phenyl-1,2,4-triazole

Following the method of Example 2, 1-benzoyl-4-(3,5-difluorobenzyl)thiosemicarbazide (5.46 g, 0.017 mole) and sodium ethoxide [from sodium (0.781 g, 0.034 mole) in ethanol (110 ml)] gave the product which was recrystallized from ethanol with melting point 188-189 °C (4.28 g, 83%).

Example 9

3,5-Difluoro-4-methoxybenzylamine

Following the method of Example 5, 3,5-difluoro-4- methoxybenzonitrile (8.0 g, 0.0473 mole) gave the product as an oil (8.0 g, 98%).

Example 10

3,5-Difluoro-4-methoxybenzylisothiocyanate

Following the method of Example 6, 3,5-difluoro-4- methoxybenzyl amine (8.0 g, 0.046 mole) gave the product as an oil (3.7 g, 37%).

Example 11

1-Benzoyl-4-(3,5-difluoro-4-methoxybenzyl)thiosemicarbazide

Following the method of Example 1, 3,5-difluoro-4- methoxybenzylisothiocyanate (3.70 g, 0.0172 mole) and benzoylhydrazine (2.34 g, 0.0172 mole) gave a solid which recrystallized from ethanol with a melting point of 165-167 °C (5.80 g, 74%).

Example 12

3-Mercapto-4-(3,5-difluoro-4-methoxybenzyl)-5-phenyl-1,2,4-triazole

Following the method of Example 2, 1-benzoyl-4-(3,5-difluoro-4-methoxybenzyl)thiosemicarbazide (3.30 g, 9.4 mmole) and sodium ethoxide [from sodium (0.432 g, 18.8 mmole) in ethanol (50 ml)] gave the product which was recrystallized from ethanol with melting point 177-178 °C (2.83 g, 90%).

Example 13

3-Mercapto-4-(3,5-difluoro-4-hydroxybenzyl)-5-phenyl-1,2,4-triazole

Boron tribromide (12.4 ml of 40% methylene chloride solution, 19.7 mmole) was added dropwise to a suspension of 3-mercapto-4-(3,5-difluoro-4-methoxybenzyl)-5-phenyl- 1,2,4-triazole and the mixture was stirred for 16 hours at 25 °C. The reaction was quenched in ice/ethyl acetate and the ethyl acetate fraction was washed with brine and dried with magnesium sulfate. The mixture was filtered and the solvent was removed under vacuum to give a solid which was recrystallized twice from ethanol to give a solid melting at 192-193 °C (1.25 g, 60%).

Example 14

1-(4-t-Butylbenzoyl)-4-benzylthiosemicarbazide

Following the method of Example 1, benzylisothiocyanate (1.38 ml, 0.0104 mole) and 4-t-butylbenzoylhydrazine (2.00 g, 0.0104 mole) gave a solid melting at 185-186 °C (2.10 g, 59%).

Example 15

3-Mercapto-4-benzyl-5-(4-t-butylphenyl)-1,2,4-triazole

Following the method of Example 2, 1-(4-t-butyl- benzoyl)-4-benzylthiosemicarbazide (2.03 g, 5.94 mmole) and sodium ethoxide [from sodium (0.273 g, 11.9 mmole) in ethanol (30 ml)] gave the product which was recrystallized from ethanol/water with melting point 191-193 °C (1.64 g, 85%).

Example 16

1-(4-t-Butylbenzoyl)-4-(3,5-difluorobenzyl)thiosemicarbazide

Following the method of Example 1, 3,5-difluorobenzylisothiocyanate (2.02 g, 0.0109 mole) and 4-t-butylbenzoylhydrazine (2.1 g, 0.0109 mole) gave a solid melting at 198-199 °C (3.57 g, 87%).

Example 17

3-Mercapto-4-(3,5-difluorobenzyl)-5-(4-t-butylphenyl)-1,2,4-triazole

Following the method of Example 2, 1-(4-t-butylbenzoyl)-4-(3,5-difluorobenzyl)thiosemicarbazide (3.45 g, 9.14 mmole) and sodium ethoxide [from sodium (0.420 g, 18.3 mmole in ethanol (50 ml)] gave the product which was recrystallized from ethanol with melting point 187-188 °C (2.15 g, 65%).

Example 18

3-Mercapto-4-phenyl-5-(4-t-butylphenyl)-1,2,4-triazole

Phenylisothiocyanate (2.40 ml, 0.02 mole) was added to a solution of 4-t-butylbenzoylhydrazine (3.85 g, 0.02 mole) and the solution was heated under reflux for one hour. A solution of sodium ethoxide [from sodium (0.92 g, 0.04 mole) in ethanol (25 ml)] was added and the solution was heated under reflux for 17 hours. Additional sodium (0.5 g) was added and the solution was heated under reflux for 24 hours. The reaction mixture was cooled in ice, acidified with 10% hydrochloric acid and the product was filtered. The solid was then triturated with a mixture of hot methanol/ethanol, cooled in ice and the product was filtered and dried with melting point 274-275 °C (3.97 g, 64%).

Example 19

3-Mercapto-4-(3-chlorophenyl)-5-(4-t-butylphenyl)-1,2,4-triazole

Following the method of Example 18, 3-chlorophenyl- isothiocyanate (2.54 g, 0.015 mole), 4-t-butylbenzoylhydrazine (2.88 g, 0.015 mole) and sodium (1.38 g, 0.06 mole) gave the crude product which was contaminated with starting material. The solid was suspended in ethanol and 10% sodium hydroxide (8 ml) was added and the solution was heated under reflux for 17 hours. The reaction mixture was cooled in ice and acidified with 10% hydrochloric acid. The product was filtered, recrystallized from ethanol/methylene chloride and dried to give a solid with a melting point of 250-251 °C (2.25 g, 44%).

Example 20

1-(4-t-Butylbenzoyl)-4-bromophenylthiosemicarbazide

Following the method of Example 1, 4-bromophenylisothiocyanate (3.21 g, 0.015 mole) and 4-t-butylbenzoylhydrazine (2.88 g, 0.015 mole) gave a solid (5.42 g, 89%).

Example 21

3-Mercapto-4-(4-bromophenyl)-5-(4-t-butylphenyl)-1,2,4-triazole

A solution of sodium ethoxide [from sodium (0.566 g, 0.0246 mole) in ethanol (20 ml)] was added to a suspension of 1-(4-t-butylbenzoyl)-4-bromophenylthiosemi carbazide (2.5 g, 6.15 mmole) and the mixture was heated under reflux for 17 hours. A 10% sodium hydroxide solution (15 ml) was added and the mixture was heated under reflux for an additional 24 hours. The reaction mixture was cooled in ice and acidified with 10% hydrochloric acid. The product was filtered, recrystallized from methanol/methylene chloride and dried to give a solid with a melting point of 256-258 °C (1.48 g, 62%).

Example 22

1-(4-t-Butylbenzoyl)-4-fluorophenylthiosemicarbazide

Following the method of Example 1, 4-fluorophenyl- isothiocyanate (2.30 g, 0.015 mole) and 4-t-butylbenzoyl- hydrazine (2.88 g, 0.015 mole) gave a solid (5.58 g, 100%).

Example 23

3-Mercapto-4-(4-fluorophenyl)-5-(4-t-butylphenyl)-1,2,4-triazole

A 10% sodium hydroxide solution (15 ml) was added to a suspension of 1-(4-t-butylbenzoyl)-4-fluorophenylthiosemicarbazide (2.5 g, 7.2 mmole) in ethanol and the mixture was heated under reflux for 17 hours. The reaction mixture was cooled in ice and acidified with 10% hydrochloric acid. The product was filtered, recrystallized from ethyl acetate/hexane and dried to give a solid with a melting point of 228-242° C (1.21 g, 51%).

Example 24

3-Phenylpropylisothiocyanate

Following the method of Example 6, 3-phenylpropyl- amine (6.76 g, 0.05 mole), thiophosgene (4.19 ml, 0.055 mole and triethylamine (15.4 mg, 0.11 mole) gave the product as an oil (6.79 g, 77%).

Example 25

3-Mercapto-4-(3-phenylpropyl)-5-(4-t-butylphenyl)-1,2,4-triazole

Following the method of Example 1, 3-phenylpropyl- isothiocyanate (2.03 g, 11.4 mmole) and 4-t-butylbenzoyl- hydrazine (2.20 g, 11.4 mmole) gave 1-(4-t-butylbenzoyl)- 4-(3-phenylpropyl)-thiosemicarbazide which was added directly as an ethanol suspension to a solution of sodium ethoxide [from sodium (0.526 g, 22.9 mmole) in ethanol (40 ml)] following the method of Example 2. The product was recrystallized twice from ethanol/water to give a solid with a melting point 153-154° C (2.99 g, 76%).

Example 26

3-Mercapto-4-(2-phenylethyl-5-(4-t-butylphenyl)-1,2,4-triazole

Phenethylisothiocyanate (2.24 ml, 0.015 mole) was added to a solution of 4-t-butylbenzoylhydrazine (2.88 g, 0.015 mole) in ethanol (40 ml) and the mixture was heated under reflux for 2 hours. A solution of sodium ethoxide [from sodium (1.03 g, 0.045 mole) in ethanol (25 ml)] was added and the mixture was heated under reflux for 17 hours. The reaction mixture was cooled in ice and acidified with 10% hydrochloric acid. The product was filtered, recrystallized from ethanol/hexane and dried to give a solid melting at 153-154° C (3.95 g, 78%).

Example 27

3-(3,5-Difluorophenyl)propylisothiocyanate

Following the method of Example 6, 3-(3,5-difluorophenyl)propylamine (4.94 g, 0.0289 mole), thiophosgene (2.242 ml, 0.0317 mole) and triethylamine (8.8 ml, 0.0635 mole) gave the product which was purified by flash silica chromatography to give an oil (4.30 g, 70%).

Example 28

3-Mercapto-4-[3-(3,5-difluorophenyl)propyl]-5-(4-t-butylphenyl)-1,2,4-triazole

3-(3,5-Difluorophenyl)propylisothiocyanate (2.13 g, 0.010 mole) was added to a solution of 4-t-butylbenzoyl- hydrazine (1.92 g, 0.010 mole) in ethanol (40 ml) and the mixture was heated under reflux for 3 hours. A solution of sodium ethoxide [from sodium (0.460 g, 0.02 mole) in ethanol (20 ml)] was added and the mixture was treated under reflux for 17 hours. The solvent was removed under vacuum and the residue was dissolved in water. The reaction mixture was cooled in ice and acidified with 10% hydrochloric acid to give a sticky solid. The aqueous solution was decanted and the solid was triturated with ethanol, filtered and recrystallized from ethanol/hexane and dried to give a solid melting at 123-124° C (0.940 g, 24%).

Example 29

3-(3,5-Difluoro-4-methoxyphenyl)propylisothiocyanate

Following the method of Example 6, 3-(3,5-difluoro- 4-methoxyphenyl)propylamine (9.03 g, 0.0448 mole), thiophosgene (3.76 ml, 0.0493 mole) and triethylamine (13.7 ml, 0.0985 mole) gave the product which was purified by flash silica chromatography to give an oil (7.30 g, 67%).

Example 30

3-Mercapto-4-[3-(3,5-difluoro-4-methoxyphenyl)propyl]-5-(4-t-butylphenyl)-1,2,4-triazole

3-(3,5-Difluoro-4-methoxyphenyl)propylisothiocyanate (7.3 g, 0.03 mole) was added to a solution of 4-t-butylbenzoylhydrazine (5.77 g, 0.03 mole) in ethanol (70 ml) and the mixture was heated under reflux for 2 hours. A solution of sodium ethoxide [from sodium 1.38 g, 0.06 mole) in ethanol (45 ml)] was added and the mixture was heated under reflux for 17 hours. The solvent was removed under vacuum and the residue was dissolved in water. The reaction mixture was cooled in ice and acidified with 10% hydrochloric acid to give a thick oil. The aqueous solution was decanted and the oil was dissolved in ethanol and the solvent was removed under vacuum. The residue was dissolved in hexane/ethyl acetate (1:1) and purified by flash silica chromatography followed by recrystallisation from ethyl acetate/hexane to give a solid melting at 173.5-174.5° C (6.53 g, 52%).

Example 31

3-Mercapto-4-[3-(3,5-difluoro-4-hydroxyphenyl)propyl]-5-(4-t-butylphenyl)-1,2,4-triazole

Boron tribromide (240 ml of 40% methylene chloride solution, 38.3 mmole) was added dropwise to a solution of 3-mercapto-4-[3-(3,5-difluoro-4-methoxyphenyl)propyl-5-(4- t-butylphenyl-1,2,4-triazole and the mixture was stirred for 16 hours at 25° C and at 35° C for 4 hours followed by an additional 16 hours at 25° C. The reaction was quenched in ice/ethyl acetate and the aqueous solution was extracted an additional 2 time with ethyl acetate. The combined ethyl acetate extracts were washed with dilute sodium bicarbonate, water and brine and dried with sodium sulfate. The solvent was removed under vacuum and the residue was dissolved in methylene chloride/methanol (19:1) and purified by flash silica chromatorgraphy to give a solid melting at 159-160° C (1.72 g, 33%).

Example 32

3-Mercapto-4-benzyl-5-methyl-1,2,4-triazole

Following the method of Example 18, benzyl isothiocyanate (3.32 ml, 0.025 mole), acethydrazide (1.95 g, 0.025 mole), and sodium ethoxide [from sodium (1.15 g, 0.05 mole) in ethanol (45 ml)] gave the product after removing the ethanol under vacuum, diluting the residue with water and acidifying with 10% hydrochloric acid. The crude product was recrystallized from ethanol to give a solid with melting point 158-160° C (1.95 g, 38%).

Example 33

3-Mercapto-4-benzyl-5-n-propyl-1,2,4-triazole

Following the method of Example 18, benzyl isothiocyanate (3.32 ml, 0.025 mole), n-butyric acid hydrazide (2.55 g, 0.025 mole), and sodium ethoxide [from sodium (1.15 g, 0.05 mole) in ethanol (40 ml)] gave the product after removing the ethanol under vacuum, diluting the residue with water and acidifying with 10% hydrochloric acid The crude product was recrystallized twice from ethanol/hexane to give a solid with melting point 127.5-128.5° C (3.08 g, 53%).

Example 34

3-Mercapto-4-benzyl-5-n-pentyl-1,2,4-triazole

Following the method of Example 18, benzyl isothiocyanate (3.32 ml, 0.025 mole), n-hexanoic acid hydrazide (3.25 g, 0.025 mole), and sodium ethoxide [from sodium (1.15 g, 0.05 mole) in ethanol (40 ml)] gave the product after removing the ethanol under vacuum, diluting the residue with water and acidifying with 10% hydrochloric acid. The crude product was recrystallized twice from ethanol/hexane to give a solid with melting point 126-127° C (4.39 g, 67%).

Example 35

3-Mercapto-4-benzyl-5-n-heptyl-1,2,4-triazole

Following the method of Example 18, benzyl isothiocyanate (3.32 ml, 0.025 mole), n-octanoic acid

hydrazide (3.96 g, 0.025 mole), and sodium ethoxide [from sodium (1.15 g, 0.05 mole) in ethanol (50 ml)] gave the product after acidifying with 10% hydrochloric acid. The crude product was recrystallized from ethanol to give a solid with melting point 120-121 °C (5.84 g, 81%).

Example 36

3-Mercapto-4-benzyl-5-n-nonyl-1,2,4-triazole

Following the method of Example 18, benzyl isothiocyanate (3.32 ml, 0.025 mole), n-decanoic acid hydrazide (4.66 g, 0.025 mole), and sodium ethoxide [from sodium (1.15 g, 0.05 mole) in ethanol (50 ml)] gave the product after acidifying with 10% hydrochloric acid. The crude product was recrystallized twice from ethanol to give a solid with melting point 116-117 °C (6.33 g, 80%).

Example 37

3-Mercapto-4-benzyl-5-cyclohexyl-1,2,4-triazole

Following the method of Example 18, benzyl isothiocyanate (3.32 ml, 0.025 mole), cyclohexane carboxylic acid hydrazide (3.59 g, 0.0252 mole), and sodium ethoxide [from sodium (1.15 g, 0.05 mole) in ethanol (50 ml)] gave the product after removing the ethanol under vacuum, diluting the residue with water and acidifying with 10% hydrochloric acid. The crude product was recrystallized three times from ethanol to give a solid with melting point 171-172 °C (3.28 g, 49%).

Example 38

3-Benzylthiosemicarbazide

Benzyl isothiocyanate (6.54 g, 0.0438 mole) was added to a solution of hydrazine monohydrate (3.32 g, 0.0657 mole) in ethanol (50 ml) and the solution was heated under reflux for 2 hours. The reaction mixture was cooled in ice and the product was filtered and washed with cold ethanol/hexane to give a solid with melting point 126-127 °C (5.50 g, 69%).

Example 39

1-t-Butylcarbonyl-4-benzylthiosemicarbazide

Trimethylacetyl chloride (3.7 ml, 0.03 mole) was added dropwise to a solution of 4-benzylthiosemicarbazide (5.44 g, 0.03 mole) in dry pyridine (40 ml) at -10 °C. The reaction was stirred at -10 °C for 15 minutes and at 25 °C for 4.75 hours. The reaction mixture was poured into crushed ice and the product was filtered and recrystallized from ethanol to give a solid with melting point 143.5-144.5 °C (6.19 g, 78%).

Example 40

3-Mercapto-4-benzyl-5-t-butyl-1,2,4-triazole

Following the method of Example 2, 1-t-butylcarbonyl- 4-benzylthiosemicarbazide (5.0 g, 0.019 mole) and sodium ethoxide [from sodium (0.866 g, 0.0377 mole) in ethanol (70 ml)] gave the product which was recrystallized from ethanol with melting point 200-201 °C 2.79 g, 60%).

Example 41

3-Mercapto-4,5-dibenzyl-1,2,4-triazole

Following the method of Example 18, benzyl isothiocyanate (3.66 ml, 0.0276 mole), phenylacetic acid hydrazide (4.14 g, 0.0276 mole), and sodium ethoxide [from sodium (1.27 g, 0.0552 mole) in ethanol (40 ml)] gave the product after removing the ethanol under vacuum, diluting the residue with water and acidifying with 10% hydrochloric acid. The crude product was recrystallized twice from ethanol to give a solid with melting point 169-170 °C (4.09 g, 53%).

Example 42

1-Phenylpropionyl-4-benzylthiosemicarbazide

Hydrocinnamoyl chloride (2.25 ml, 0.0152 mole) was added dropwise to a solution of 4-benzylthiosemicarbazide (2.75 g, 0.0152 mole) in dry pyridine (25 ml) at -10 °C. The reaction was stirred at -10 °C for 10 minutes and then at 25 °C. An additional 0.5 ml of hydrocinnamoyl chloride was added and the reaction mixture was stirred for 17 hours. Another 1.0 ml of the acid chloride was added and the reaction mixture was poured into crushed ice and the product was filtered and triturated twice with ethanol to give a solid with melting point 178-180 °C (3.48 g, 73%).

Example 43

3-Mercapto-4-benzyl-5-phenethyl-1,2,4-triazole

Following the method of Example 2, 1-phenylpropionyl- 4-benzylthiosemicarbazide (3.41 g, 0.0109 mole) and sodium ethoxide [from sodium (0.50 g, 0.0218 mole) in ethanol (50 ml)] gave the product which was recrystallized from ethanol, then ethyl acetate and finally ethyl acetate/ethanol with melting point 189-190 °C (1.85 g, 60%).

Example 44

3-Mercapto-4-benzyl-5-(4-methoxyphenyl)-1,2,4-triazole

Following the method of Example 18, benzyl isothiocyanate (3.32 ml, 0.025 mole), 4-methoxybenzhydrazide (4.14 g, 0.025 mole), and sodium ethoxide [from sodium (1.15 g, 0.05 mole) in ethanol (80 ml)] gave the product after removing the ethanol under vacuum, diluting the residue with water and acidifying with 10% hydrochloric acid. The crude product was recrystallized twice from ethanol to give a solid with melting point 202-203 °C (4.16 g, 56%).

## Example 45

3-Mercapto-4-benzyl-5-(3,4,5-trimethoxyphenyl)-1,2,4-triazole

Following the method of Example 18, benzyl isothiocyanate (3.32 ml, 0.025 mole), 3,4,5-trimethoxybenzhydrazide (5.66 g, 0.025 mole), and sodium ethoxide [from sodium (1.15 g, 0.05 mole) in ethanol (50 ml)] gave the product after removing the ethanol under vacuum, diluting the residue with water and acidifying with 10% hydrochloric acid. The crude product was recrystallized twice from ethanol, dissolved in dilute sodium hydroxide, filtered and reacidified with 10 % hydrochloric acid. The solid was filtered and recrystallized twice from ethanol to give a solid with melting point 177-178 °C (3.39 g, 38%).

## Example 46

3-Mercapto-4-benzyl-5-(4-chlorophenyl)-1,2,4-triazole

Following the method of Example 18, benzyl isothiocyanate (3.32 ml, 0.025 mole), 4-chlorobenzhydrazide (4.26 g, 0.025 mole), and sodium ethoxide [from sodium (1.15 g, 0.05 mole) in ethanol (80 ml)] gave the product after removing the ethanol under vacuum, diluting the residue with water and acidifying with 10% hydrochloric acid. The crude product was recrystallized from ethanol to give a solid with melting point 197-198 °C (4.58 g, 61%).

## Example 47

3-Mercapto-4-benzyl-5-(4-bromophenyl)-1,2,4-triazole

Following the method of Example 18, benzyl isothiocyanate (1.99 ml, 0.015 mole), 4-bromobenzhydrazide (3.23 g, 0.015 mole), and sodium ethoxide [from sodium (0.690 g, 0.03 mole) in ethanol (25 ml)] gave the product after removing the ethanol under vacuum, diluting the residue with water and acidifying with 10% hydrochloric acid. The crude product was recrystallized twice from ethanol to give a solid with melting point 213-214 °C (2.81 g, 54%).

## Example 48

3-Mercapto-4-benzyl-5-(3-bromophenyl)-1,2,4-triazole

Following the method of Example 18, benzyl isothiocyanate (1.99 ml, 0.015 mole), 3-bromobenzhydrazide (3.23 g, 0.015 mole), and sodium ethoxide [from sodium (0.690 g, 0.03 mole) in ethanol (25 ml)] gave the product after removing the ethanol under vacuum, diluting the residue with water and acidifying with 10% hydrochloric acid. The crude product was recrystallized twice from ethanol to gave a solid with melting point 176-177 °C (3.68 g, 71%).

The following lettered examples describe preparation of selected compounds used in preparing compounds of structure (I).

## Example A

3,5-Difluorobenzaldehyde

A mixture of 3,5-difluorobenzonitrile (15.0 g, 0.11 mole) and Raney catalyst powder (15 g) in 90% formic acid (150 ml) was stirred under reflux for 2.5 hours and the catalyst was filtered and washed with hot water and hexane. The hexane layer was separated and the aqueous solution was extracted two more times with hexane. The combined hexane extracts were washed with water and brine, dried and the solvent was removed to give an oil (8.51 g, 56%).

Example B

3,5-Difluorocinnamic acid

A mixture of 3,5-difluorobenzaldehyde (8.5 g, 0.0598 mole), malonic acid (9.29 g, 0.0893 mole), pyridine (3.2 ml) and piperidine (0.15 ml) was heated for 1.5 hours at 100 °C and 3 hours at 150 °C. The reaction mixture was cooled to room temperature and the resulting solid was triturated with 10% hydrochloric acid and filtered. The product then was triturated with ethanol, filtered and dried to give a solid with melting point 199-201 °C (8.12 g, 74%).

Example C

3-(3,5-Difluorophenyl)propionic acid

A suspension of 10% palladium on carbon (1.5 g) in ethyl acetate was added to a solution of 3,5-difluorocinnamic acid (8.12 g, 0.0441 mole) in tetrahydrofuran (100 ml) and the mixture was shaken under a hydrogen atmosphere (50 pounds) for 1 hour. The catalyst was filtered and the solvent was removed under vacuum to give the product as a solid (8.25 g, 100%).

Example D

3-(3,5-Difluorophenyl)propanol

A solution of 1M borane (97 ml, 0.097 mole) in tetrahydrofuran was added to a solution of 3-(3,5-difluorophenyl)propionic acid (8.21 g, 0.0441 mole) in tetrahydrofuran (75 ml) at 0 °C and the solution was stirred at 25° for 17 hours. The reaction mixture was cooled in ice, and methanol was slowly added to destroy excess borane. The solvent was removed under vacuum and the residue was dissolved in ether and the mixture was filtered. The ether solution was washed with water and brine and then dried over sodium sulfate. The solvent was removed to give the product as an oil (8.37 g, 100%)

Example E

3-(3,5-Difluorophenyl)propyl azide

p-Toluenesulfonyl chloride (18.5 g, 0.0972 mole) was added to a solution of 3-(3,5-difluorophenyl)-propanol (8.37 g, 0.0486 mole) in pyridine (75 ml) at 0 °C. The reaction mixture was stirred at 0 °C for 20 minutes and at 25 °C for 2 hours and then kept at 4 °C for 17 hours. The mixture was poured into an ice/water mixture and extracted with 3 portions of ether. The ether solution was washed with several

portions of cold 1N hydrochloric acid followed by water and then brine. The solution was dried over sodium sulfate and the solvent was removed to give the crude tosylate as an oil. The oil was taken up in dimethylformamide (75 ml) and sodium azide (6.32 g, 0.0972 mole) was added and the mixture was stirred for 17 hours under an argon atmosphere. The reaction mixture was quenched in ice water and then extracted with 3 portions of ethyl acetate. The solution was washed with cold 1N hydrochloric acid followed by water and brine and then dried over sodium sulfate. The solvent was removed under vacuum and the resulting oil was dissolved in hexane/ethyl acetate (9:1) and purified by flash silica chromatography to give the product as an oil (6.01 g, 63%).

## Example F

### 3-(3,5-Difluorophenyl)propylamine

A solution of 3-(3,5-difluorophenyl)propyl azide in methanol (75 ml) and Raney nickel was shaken in a hydrogen atmosphere (50 pounds) for 5.5 hours. The catalyst was filtered and the solvent was removed under vacuum to give the product as an oil (4.94 g, 98%).

## Example G

### 3,5-Difluoro-4-methoxybenzaldehyde

A mixture of 3,5-difluoro-4-methoxybenzonitrile (18.0 g, 0.106 mole) and Raney catalyst powder (18 g) in 90% formic acid (180 ml) was stirred under reflux for 3 hours and the catalyst filtered and washed with hot water and hexane. The hexane layer was separated and the aqueous solution was extracted an additional four times with hexane. The combined hexane extracts were washed with water and brine, dried and the solvent was removed to give a solid (16.5 g, 90%).

## Example H

### 3,5-Difluoro-4-methoxycinnamic acid

A mixture of 3,5-difluoro-4-methoxybenzaldehyde (16.5 g, 0.0959 mole), malonic acid (15.0 g, 0.144 mole), pyridine (5.3 ml, 0.065 mole) and piperidine (0.26 ml, 2.6 mmole) was heated for 1 hour at 100°C and 4 hours at 150°C. The reaction mixture was cooled to room temperature and the resulting solid was triturated with 10% hydrochloric acid and filtered. The product was then triturated with ethanol, filtered and dried to give a solid with melting point 211-213°C (17.3 g, 84%).

## Example I

### 3-(3,5-Difluoro-4-methoxyphenyl)propionic acid

A suspension of 10% palladium in carbon (1.5 g) in ethyl acetate was added to a solution of 3,5-difluoro-4-methoxycinnamic acid (17.3 g, 0.0808 mole) in tetrahydrofuran (150 ml) and the mixture was shaken under a hydrogen atmosphere (50 pounds) for 1 hour. The catalyst was filtered and the solvent was removed under vacuum to give the product as a solid (17.5 g, 100%).

Example J

3-(3.5-Difluoro-4-methoxyphenyl)propanol

A solution of 1M borane (178 ml, 0.178 mole) in tetrahydrofuran was added to a solution of 3-(3,5-difluoro-4-methoxyphenyl)propionic acid (17.5 g, 0.0808 mole) in tetrahydrofuran (125 ml) at 0° C and the solution was stirred at 25° for 17 hours. The reaction mixture was cooled in ice and methanol was slowly added to destroy excess borane. The solvent was removed under vacuum and the residue was dissolved in ether and the mixture was filtered. The ether solution was washed with water and brine and then dried over sodium sulfate. The solvent was removed to give the product as an oil (16.7 g, 100%).

Example K

3-(3,5-Difluorophenyl)propyl azide

p-Toluenesulfonyl chloride (17.9 g, 0.0939 mole) was added to a solution of 3-(3,5-difluoro-4-methoxyphenyl)propanol (9.49 g, 0.0469 mole) in pyridine (120 ml) at 0° C. The reaction mixture was stirred at 0° C for 6 hours and then kept at -10° C for 2 days. The mixture was poured into an ice/water mixture and extracted with 3 portions of ether. The ether solution was washed with several portions of cold 1N hydrochloric acid followed by water and then brine. The solution was dried over sodium sulfate and the solvent was removed to give the crude tosylate as an oil. The oil was taken up in dimethylformamide (80 ml) and sodium azide (6.10 g, 0.0939 mole) was added and the mixture was stirred for 17 hours under an argon atmosphere. The reaction mixture was quenched in ice water and then extracted with 3 portions of ethyl acetate. The solution was washed with water and brine and then dried over sodium sulfate. The solvent was removed under vacuum to give the product as an oil (11.3 g, 100%).

Example L

3-(3.5-Difluoro-4-methoxyphenyl)propylamine

A solution of 3-(3,5-difluoro-4-methoxyphenyl)propyl azide in methanol (110 ml) and Raney nickel was shaken in a hydrogen atmosphere (50 pounds) for 3 hours. The catalyst was filtered and the solvent was removed under vacuum to give the product as an oil (9.01 g, 95%).

Example M

n-Hexanoic acid hydrazide

A solution of ethyl hexanoate (14.4 g, 0.10 mole) and hydrazine monohydrate (7.35 ml, 0.15 mole) in ethanol (75 ml) was heated under reflux for 17 hours. The solvent was removed under vacuum and the residual oil was triturated with hexane with ice cooling. The product was filtered and recrystallized from ether to give a solid with melting point 70.5-71.5° C (6.10 g, 47%).

Example N

n-octanoic acid hydrazide

Following the method of Example M, ethyl octanoate (17.2 g, 0.10 mole) and hydrazine monohydrate (7.35 ml, 0.15 mole) in ethanol (75 ml) gave the product by triturating the residual oil with ether to give a solid with melting point 85-87° C (5.50 g, 35%).

Example O

n-Decanoic acid hydrazide

Following the method of Example M ethyl decanoate (20.0 g, 0.10 mole) and hydrazine monohydrate (7.25 ml, 0.15 mole) in ethanol (75 ml) gave the product by removing about half the solvent under vacuum, cooling in ice and filtering off the solid. The crude product was recrystallized from ethanol/hexane to give a solid with melting point 95-96.5° C (8.56 g, 46%).

Example P

Cyclohexane carboxylic acid hydrazide

Following the method of Example M, methyl cyclohexane carboxylate (14.2 g, 0.10 mole) and hydrazine monohydrate (7.25 ml, 0.15 mole) in ethanol (100 ml) gave the product by triturating the residual oil with ether/hexane and recrystallizing with ethyl acetate/hexane to give a solid with melting point 149-153° C (3.59 g, 25%).

Example Q

Phenylacetic acid hydrazide

Following the method of Example M, ethyl phenylacetate (16.4 g, 0.10 mole) and hydrazine monohydrate (7.35 ml, 0.15 mole) in ethanol (200 ml) gave the product by triturating the residual oil with ether and recrystallizing twice from ethanol to give a solid with melting point 110-112° C (4.14 g, 28%).

Example R

3-Bromobenzoic acid hydrazide

Following the method of Example M, ethyl-3-bromobenzoate (22.9 g, 0.10 mole) and hydrazine monohydrate (7.35 ml 0.15 mole) in ethanol (75 ml) gave the product by diluting the reaction mixture with ether and filtering off the product to give a solid with melting point 153-154.5° C (14.7 g, 68%).

Example 49

An oral dosage form for administering the presently invented compounds is produced by screening, mixing, and filling into hard gelatin capsules the ingredients in the proportions shown in Table II, below.

Table II

| Ingredients | Amounts |
|---|---|
| 3-Mercapto-4-(3,5-difluorobenzyl)-5-phenyl-1,2,4-triazole | 50 mg |
| magnesium stearate | 5 mg |
| lactose | 75 mg |

Example 50

The sucrose, calcium sulfate dihydrate, and structure (I) compound shown in Table III below, are mixed and granulated in the proportions shown with a 10% gelatin solution. The wet granules are screened, dried, mixed with the starch, talc and stearic acid, screened and compressed into a tablet.

Table III

| Ingredients | Amounts |
|---|---|
| 3-Mercapto-4-benzyl-5-(4-t-butylphenyl)-1,2,4-triazole | 100 mg |
| calcium sulfate dihydrate | 150 mg |
| sucrose | 20 mg |
| Starch | 10 mg |
| talc | 5 mg |
| stearic acid | 3 mg |

Example 51

3-Mercapto-4,5-dibenzyl-1,2,4-triazole, 75 mg, is dispersed in 25 ml of normal saline to prepare an injectable preparation.

Contemplated equivalents of compounds of structure (I) are compounds that upon administration to mammals, including humans, are metabolized to compounds of structure (I) or metabolized to any active metabolites of compounds of structure (I) at a sufficient rate and in sufficient amounts to produce the physiological activity of compounds of structure (I). Such compounds also would be included in the invented pharmaceutical compositions and used in the invented methods.

While the preferred embodiments of the invention are illustrated by the above, the invention is not limited to the precise instructions herein disclosed and that the right to all modifications coming within the scope of the following claims is reserved.

**Claims**

1. A compound of formula (I)

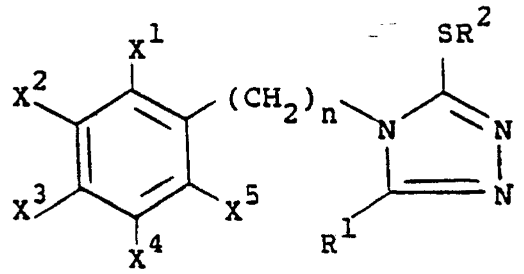

(I)

in which,
n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, OH, $CH_2OH$, $CO_2H$, or $CO_2C_pH_{2p+1}$ wherein p is 1 to 4;
$R^1$ is phenyl substituted by $X^1$ to $X^5$, $C_{1-4}$alkyl, $C_{3-6}$cycloalkyl, or an aryl$C_{1-4}$alkyl group substituted by $X^1$ to $X^5$;
$R^2$ is hydrogen, $C_{1-4}$alkyl or $(CH_2)_m$-$CO_2R^3$;
m is 0 to 5; and
$R^3$ is H or $C_{1-4}$alkyl; or
a pharmaceutically acceptable salt thereof provided that :

(i) when n is O, $R^2$ is hydrogen and $X^1$ to $X^5$ are hydrogen, $R^1$ is other than phenyl or phenyl substituted by OH, $C_{1-6}$alkoxy, halogen;

(ii) when n is O, $R^2$ is hydrogen, $X^1$ is $C_{1-6}$alkyl or $C_{1-6}$alkoxy and $X^2$ to $X^5$ are hydrogen, $R^1$ is other than phenyl or phenyl substituted by $C_{1-6}$alkyl, $C_{1-6}$alkoxy, hydroxy, nitro or halogen;

(iii) when n is O, $R^2$ is hydrogen, $X^2$ is $C_{1-6}$alkyl or halogen and $X^1$ and $X^3$ to $X^5$ are hydrogen, $R^1$ is other than phenyl or phenyl substituted by $C_{1-6}$alkyl, $C_{1-6}$alkoxy, hydroxy or halogen;

(iv) when n is O, $R^2$ is hydrogen, $X^1$, $X^2$ and $X^4$, $X^5$ are hydrogen and $X^3$ is $C_{1-6}$alkyl, halogen or $C_{1-6}$alkoxy, $R^1$ is other than phenyl or phenyl substituted by $C_{1-6}$alkoxy, hydroxy, halogen or nitro.

(v) when n is O, $R^2$ is hydrogen, $X^4$ and $X^5$ are hydrogen, one of $X^1$ and $X^2$ is hydrogen and the other is $C_{1-6}$alkyl and $X^3$ is $C_{1-6}$alkyl, $R^1$ is other than a phenyl group substituted by three $C_{1-6}$alkoxy groups;

(vi) when n is O, $R^2$ is hydrogen, $X^1$, $X^4$ and $X^5$ are hydrogen and $X^2$ and $X^3$ are halogen, $R^1$ is other than a phenyl group substituted by hydroxy or halogen; and

(vii) when n is 1, $R^2$ is hydrogen and $X^1$ to $X^5$ are all hydrogen, $R^1$ is other than phenyl, or a phenyl group monosubstituted by methyl, methoxy, chloro or nitro, or a phenyl group disubstituted by chloro.

2. A compound according to claim 1 in which n is 0 or 1.

3. A compound according to claim 1 or 2 in which one or two of $X^1$ to $X^5$ is halogen.

4. A compound according to claim 1 or 2 in which $X^2$ or $X^4$ is halogen or $X^4$ and $X^2$ are halogen.

5. A compound according to claim 1 or 2 in which $X^2$ and $X^4$ are halogen and $X^3$ is $C_{1-6}$alkoxy.

6. A compound according to claim 1 which is:
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4 triazole,
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2,4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,

3-mercapto-4-[3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n-propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,
3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto 4-benzyl-5-(3,4,5-trimethoxyphenyl)-1,2,4-triazole,
3-mercapto-4-benzyl-5-(4-bromophenyl)-1,2,4-triazole, or
3-mercapto-4-benzyl-5-(3-bromophenyl)-1,2,4-triazole,
or a pharmaceutically acceptable salt thereof.

7. A compound of formula (Ib)

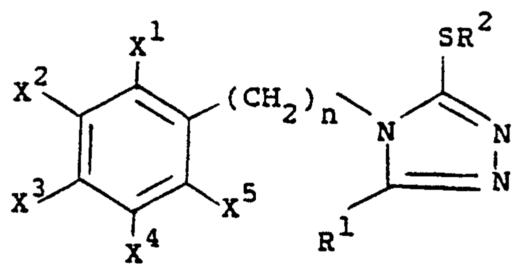

(Ib)

in which,
n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, OH, $CH_2OH$, $CO_2H$, or $CO_2C_pH_{2p+1}$ wherein p is 1 to 4;
$R^1$ is phenyl substituted by $X^1$to $X^5$, $C_{1-4}$alkyl, $C_{3-6}$cycloalkyl, or an aryl$C_{1-4}$alkyl group substituted by $X^1$ to $X^5$;
$R^2$ is hydrogen, $C_{1-4}$alkyl, or $(CH_2)_m$-$CO_2R^3$;
m is 0 to 5; and
$R^3$ is H or $C_{1-4}$alkyl;
or a pharmaceutically acceptable salt thereof, for use as a medicament.

8. A compound according to claim 7 which is :
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole,
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-benzyl-5-phenyl-1,2,4-trazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2,4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,
3-mercapto-4-[3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n-propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,

3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(4-methoxyphenyl)-1,2,4-triazole,
3-mercapto-4-benzyl-5-(3,4,5-trimethoxyphenyl)-1,2,4-triazole,
3-mercapto-4-benzyl-5-(4-chlorophenyl)-1,2,4-triazole,
3-mercapto-4-benzyl-5-(4-bromophenyl)-1,2,4-triazole, or
3-mercapto-4-benzyl-5-(3-bromophenyl)-1,2,4-triazole,
or a pharmaceutically acceptable salt thereof.

9. A pharmaceutical composition which comprises a compound according to any one of claims 1 to 8 and a pharmaceutically acceptable carrier.

10. The use of a compound of formula (Ib) as defined in claim 7 in the manufacture of a medicament for inhibiting dopamine-β-hydroxylase activity.

11. A process for preparing a compound of formula (I) or a pharmaceutically acceptable salt thereof as defined in claim 1 which comprises cyclization of a compound of formula (II)

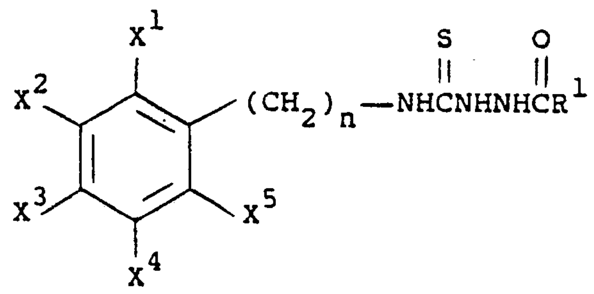

(II)

in which $X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, $CH_2OC_{1-6}$alkyl, or $CO_2C_{1-6}$alkyl; and n and $R^1$ are as defined in claim 1 and optionally thereafter :

(1) converting a group $X^1$ to $X^5$ into a OH, $CH_2OH$ or $CO_2H$ group,

(2) converting a compound of formula (I) in which $R^2$ is hydrogen to one in which $R^2$ is $C_{1-4}$alkyl or $(CH_2)_mCO_2R^3$ in which m and $R^3$ are as defined in claim 1, and

(3) forming a pharmaceutically acceptable salt.

12. A compound of formula (II)

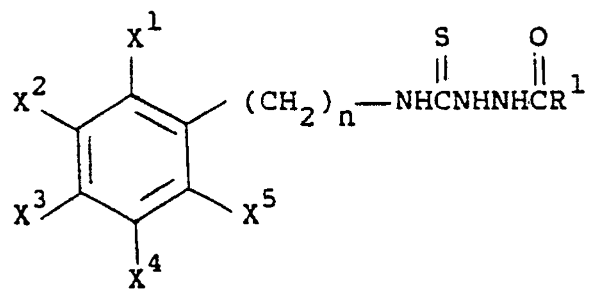

(II)

in which,
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, $CH_2OC_{1-6}$alkyl, or $CO_2C_{1-6}$alkyl;
and n and $R^1$ are as defined in claim 1.

Claims for the following Contracting State : ES

1. A process for the preparation of a compound of formula (I)

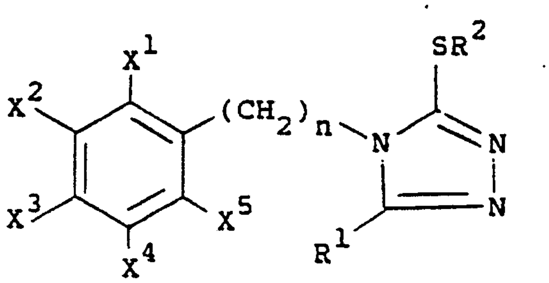

(I)

in which,
n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, OH, $CH_2OH$, $CO_2H$, or $CO_2C_pH_{2p+1}$ wherein p is 1 to 4;
$R^1$ is phenyl substituted by $X^1$ to $X^5$, $C_{1-4}$alkyl, $C_{1-6}$cycloalkyl, or an aryl$C_{1-4}$alkyl group substituted by $X^1$ to $X^5$;
$R^2$ is hydrogen, $C_{1-4}$alkyl or $(CH_2)_m$-$CO_2R^3$;
m is 0 to 5; and
$R^3$ is H or $C_{1-4}$alkyl; or
a pharmaceutically acceptable salt thereof provided that :
(i) when n is O, $R^2$ is hydrogen and $X^1$ to $X^5$ are hydrogen, $R^1$ is other than phenyl or phenyl substituted by OH, $C_{1-6}$alkoxy, halogen;
(ii) when n is O, $R^2$ is hydrogen, $X^1$ is $C_{1-6}$alkyl or $C_{1-6}$alkoxy and $X^2$ to $X^5$ are hydrogen, $R^1$ is other than phenyl or phenyl substituted by $C_{1-6}$alkyl, $C_{1-6}$alkoxy, hydroxy, nitro or halogen;
(iii) when n is O, $R^2$ is hydrogen, $X^2$ is $C_{3-6}$alkyl or halogen and X and $X^3$ to $X^5$ are hydrogen, $R^1$ is other than phenyl or phenyl substituted by $C_{1-6}$alkyl, $C_{1-6}$alkoxy, hydroxy or halogen;
(iv) when n is O, $R^2$ is hydrogen, $X^1$, $X^2$ and $X^4$, $X^5$ are hydrogen and $X^3$ is $C_{1-6}$alkyl, halogen or $C_{1-6}$alkoxy, $R^1$ is other than phenyl or phenyl substituted by $C_{1-6}$alkoxy, hydroxy, halogen or nitro.
(v) when n is O, $R^2$ is hydrogen, $X^4$ and $X^5$ are hydrogen, one of $X^1$ and $X^2$ is hydrogen and the other is $C_{1-6}$alkyl and $X^3$ is $C_{1-6}$alkyl, $R^1$ is other than a phenyl group substituted by three $C_{1-6}$alkoxy groups;
(vi) when n is O, $R^2$ is hydrogen, $X^1$, $X^4$ and $X^5$ are hydrogen and $X^2$ and $X^3$ are halogen, $R^1$ is other than a phenyl group substituted by hydroxy or halogen; and
(vii) when n is 1, $R^2$ is hydrogen and $X^1$ to $X^5$ are all hydrogen, $R^1$ is other than phenyl, or a phenyl group monosubstituted by methyl, methoxy, chloro or nitro, or a phenyl group disubstituted by chloro;
which process comprises cyclization of a compound of formula (II)

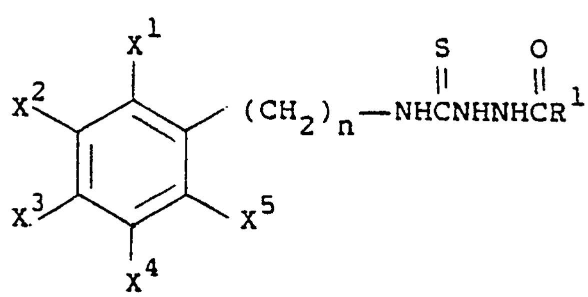

(II)

in which $X^1$ to $X^5$ are any accessible combination of hydrogen, halogen, $C_{1-6}$alkyl, $C_{1-6}$alkoxy, cyano, nitro $SONH_2$, $SO_2NH_2$, $SO_2CH_3$, $SO_2CH_2F$, $SO_2CHF_2$, $SO_2CF_3$, $CF_3$, CHO, $CH_2OC_{1-6}$alkyl, or $CO_2C_{1-6}$alkyl; and n and $R^1$ are as hereinbefore defined and optionally thereafter :

(1) converting a group $X^1$ to $X^5$ into a OH, $CH_2OH$ or $CO_2H$ group,

(2) converting a compound of formula (I) in which $R^2$ is hydrogen to one in which $R^2$ is $C_{1-4}$alkyl or $(CH_2)_mCO_2R^3$ in which m and $R^3$ are as hereinbefore, and

(3) forming a pharmaceutically acceptable salt.

2. A process according to claim 1 for preparing a compound in which n is 0 or 1.

3. A process according to claim 1 or 2 for preparing a compound in which one or two of $X^1$ to $X^5$ is halogen.

4. A process according to claim 1 or 2 for preparing a compound in which $X^2$ or $X^4$ is halogen or $X^4$ and $X^2$ are halogen.

5. A process according to claim 1 or 2 for preparing a compound in which $X^2$ and $X^4$ are halogen and $X^3$ is $C_{1-6}$alkoxy.

6. A process according to claim 1 for preparing a compound which is:
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole,
3-mercapto-4-benzyl-5-(4-t-butylphenyl)-1,2,4-triazole,
3-mercapto-4-(3,5-difluorobenzyl)-5-(4-t-butylphenyl)1,2,4-triazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2 4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2 4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,
3-mercapto-4-[3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,
3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(3,4,5-trimethoxyphenyl)-1,2,4-triazole,
3-mercapto-4-benzyl-5-(4-bromophenyl)-1,2 4-triazole, or
3-mercapto-4-benzyl-5-(3-bromophenyl)-1,2,4-triazole,
or a pharmaceutically acceptable salt thereof.

7. A process for preparing a pharmaceutical composition which comprises bringing into association a pharmaceutically acceptable carrier and a compound of formula (Ib)

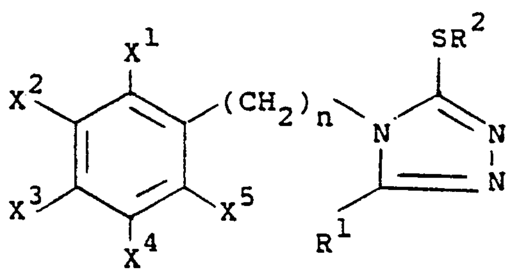

(Ib)

in which,
n is 0 to 5;
$X^1$ to $X^5$ are any accessible combination of hydrogen, halogen, $C_{1-6}$alkyl, $C_{1-6}$alkoxy cyano, nitro, $SONH_2$, $SO_2NH_2$, $SO_2CH_3$, $SO_2CH_2F$, $SO_2CHF_2$, $SO_2CF_3$, $CF_3$, CHO, OH, $CH_2OH$, $CO_2H$ or $CO_2C_pH_{2p+1}$ wherein p is 1 to 4;
$R^1$ is phenyl substituted by $X^1$ to $X^5$, $C_{1-4}$alkyl, $C_{3-6}$cycloalkyl, or an aryl$C_{1-4}$alkyl group substituted by $X^1$ to $X^5$;
$R^2$ is hydrogen, $C_{1-4}$alkyl, or $(CH_2)_m$-$CO_2R^3$;
m is 0 to 5; and
$R^3$ is H or $C_{1-4}$alkyl;
or a pharmaceutically acceptable salt thereof.

8. A process according to claim 7 wherein a compound of formula (1b) is :
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole,
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-benzyl-5-phenyl-1,2,4-trazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2,4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,
3-mercapto-4-[3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n-propyl-1,2,4-triazole,
3-mercapto-4 benzyl-5-n-pentyl-1,2,4-triazole,
3-mercapto-4-benzyl-5-n-heptyl-1,2,4-triazole,
3 mercapto-4-benzyl-5-n-nonyl-1,2,4-triazole,
3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(4-methoxyphenyl)-1,2 4-triazole,
3-mercapto-4-benzyl-5-(3,4,5-trimethoxyphenyl)-1,2,4-triazole,
3-mercapto-4-benzyl-5-(4-chlorophenyl)-1,2,4-triazole,
3-mercapto-4-benzyl-5-(4-bromophenyl)-1,2,4-triazole, or
3-mercapto-4-benzyl-5-(3-bromophenyl)-1,2,4-triazole,
or a pharmaceutically acceptable salt thereof.

9. The use of a compound of formula (Ib) as defined in claim 7 in the manufacture of a medicament for inhibiting dopamine-β-hydroxylase activity.

10. A process for preparing a compound of formula (II)

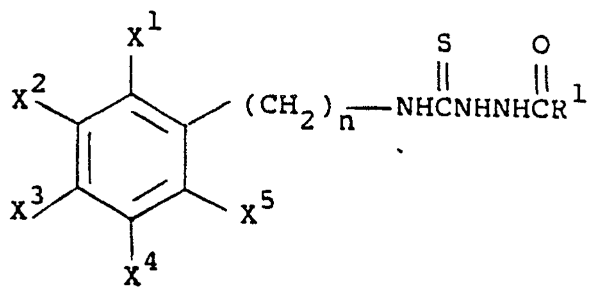

(II)

in which,
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, $CH_2OC_{1-6}$alkyl, or $CO_2C_{1-6}$alkyl;
and n and $R^1$ are as defined in claim 1;
which comprises reacting a compound of formula (III) with a compound of formula (IV) :

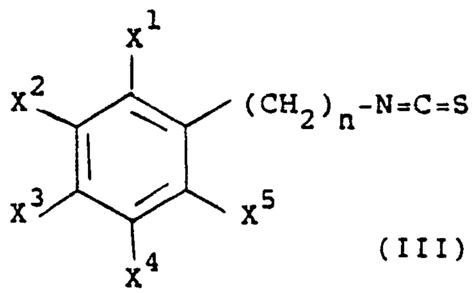

(III)

$R^1CONHNH_2$ (IV)

in which,

$X^1$ to $X^5$, n and $R^1$ are as hereinbefore defined.

Claims for the following Contrating State : GR

1. A process for the preparation of a compound of formula (I)

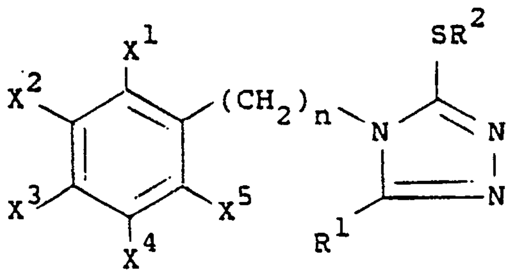

(I)

in which,
n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, OH, $CH_2OH$, $CO_2H$, or $CO_2C_pH_{2p+1}$ wherein p is 1 to 4;
$R^1$ is phenyl substituted by $X^1$ to $X^5$, $C_{1-4}$alkyl, $C_{3-6}$cycloalkyl, or an aryl$C_{1-4}$alkyl group substituted by $X^1$ to $X^5$;
$R^2$ is hydrogen, $C_{1-4}$alkyl or $(CH_2)_m$-$CO_2R^3$;
m is 0 to 5; and
$R^3$ is H or $C_{1-4}$alkyl; or
a pharmaceutically acceptable salt thereof provided that :
(i) when n is O, $R^2$ is hydrogen and $X^1$ to $X^5$ are hydrogen, $R^1$ is other than phenyl or phenyl substituted by OH, $C_{1-6}$alkoxy, halogen;
(ii) when n is O, $R^2$ is hydrogen, $X^1$ is $C_{1-6}$alkyl or $C_{1-6}$alkoxy and $X^2$ to $X^5$ are hydrogen, $R^1$ is other than phenyl or phenyl substituted by $C_{1-6}$alkyl, $C_{1-6}$alkoxy, hydroxy, nitro or halogen;
(iii) when n is O, $R^2$ is hydrogen, $X^2$ is $C_{1-6}$alkyl or halogen and $X^1$ and $X^3$ to $X^5$ are hydrogen, $R^1$ is other than phenyl or phenyl substituted by $C_{1-6}$alkyl, $C_{1-6}$alkoxy, hydroxy or halogen;
(iv) when n is O, $R^2$ is hydrogen, $X^1$, $X^2$ and $X^4$, $X^5$ are hydrogen and $X^3$ is $C_{1-6}$alkyl, halogen or $C_{1-6}$alkoxy, $R^1$ is other than phenyl or phenyl substituted by $C_{1-6}$alkoxy, hydroxy, halogen or nitro.
(v) when n is O, $R^2$ is hydrogen, $X^4$ and $X^5$ are hydrogen, one of $X^1$ and $X^2$ is hydrogen and the other is $C_{1-6}$alkyl and $X^3$ is $C_{1-6}$alkyl, $R^1$ is other than a phenyl group substituted by three $C_{1-6}$alkoxy groups;
(vi) when n is O, $R^2$ is hydrogen, $X^1$, $X^4$ and $X^5$ are hydrogen and $X^2$ and $X^3$ are halogen, $R^1$ is other than a phenyl group substituted by hydroxy or halogen; and
(vii) when n is 1, $R^2$ is hydrogen and $X^1$ to $X^5$ are all hydrogen, $R^1$ is other than phenyl, or a phenyl group monosubstituted by methyl, methoxy, chloro or nitro, or a phenyl group disubstituted by chloro;
which process comprises cyclization of a compound of formula (II)

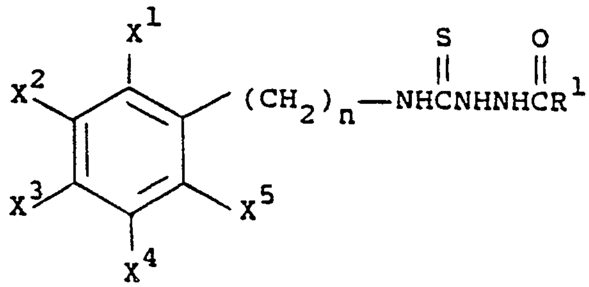

(II)

in which $X^1$ to $X^5$ are any accessible combination of hydrogen, halogen, $C_{1-6}$alkyl, $C_{1-6}$alkoxy cyano, nitro, $SONH_2$, $SO_2NH_2$, $SO_2CH_3$, $SO_2CH_2F$, $SO_2CHF_2$, $SO_2CF_3$, $CF_3$, CHO, $CH_2OC_{1-6}$alkyl, or $CO_2C_{1-6}$alkyl; and n and $R^1$ are as hereinbefore defined and optionally thereafter :

(1) converting a group $X^1$ to $X^5$ into a OH, $CH_2OH$ or $CO_2H$ group,

(2) converting a compound of formula (I) in which $R^2$ is hydrogen to one in which $R^2$ is $C_{1-4}$alkyl or $(CH_2)_mCO_2R^3$ in which m and $R^3$ are as hereinbefore defined, and

(3) forming a pharmaceutically acceptable salt.

2. A process according to claim 1 for preparing a compound in which n is 0 or 1.

3. A process according to claim 1 or 2 for preparing a compound in which one or two of $X^1$ to $X^5$ is halogen.

4. A process according to claim 1 or 2 for preparing a compound in which $X^2$ or $X^4$ is halogen or $X^4$ and $X^2$ are halogen.

5. A process according to claim 1 or 2 for preparing a compound in which $X^2$ and $X^4$ are halogen and $X^3$ is $C_{1-6}$alkoxy.

6. A process according to claim 1 for preparing a compound which is:
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t butylphenyl)-1,2,4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,
3-mercapto-4-[3-(3,5-difluoro-4-hydroxyphenyl)propyl]-5-(4-t-butylphenyl)-1,2,4-triazole,
3-mercapto-4-benzyl-5-methyl-1,2,4-triazole,
3-mercapto-4-benzyl-5-n-propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,
3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(3,4,5-trimethoxyphenyl)-1,2,4-triazole,
3-mercapto-4-benzyl-5-(4-bromophenyl)-1,2,4-triazole, or
3-mercapto-4-benzyl-5-(3-bromophenyl)-1,2,4-triazole,
or a pharmaceutically acceptable salt thereof.

7. A process for preparing a pharmaceutical composition which comprises bringing into association a pharmaceutically acceptable carrier and a compound of formula (Ib)

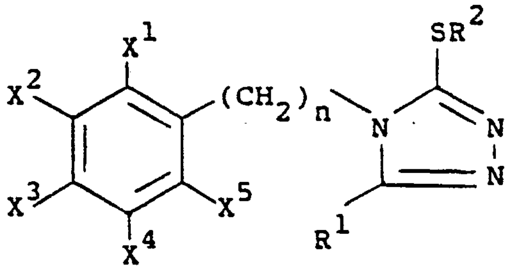

(Ib)

in which,
n is 0 to 5;
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$ CHO, OH, $CH_2OH$, or $CO_2H$, $CO_2C_pH_{2p+1}$ wherein p is 1 to 4;
$R^1$ is phenyl substituted by $X^1$ to $X^5$, $C_{1-4}$alkyl, $C_{3-6}$cycloalkyl, or an aryl$C_{1-4}$alkyl group substituted by $X^1$ to $X^5$;
$R^2$ is hydrogen, $C_{1-4}$alkyl, or $(CH_2)_m$-$CO_2R^3$;
m is 0 to 5; and
$R^3$ is H or $C_{1-4}$alkyl;
or a pharmaceutically acceptable salt thereof.

8. A process according to claim 7 wherein a compound of formula (1b) is :
3-mercapto-4-(3,5-difluorobenzyl)-5-phenyl-1,2,4-triazole,
3-mercapto-4-(3,5-difluoro-4-methoxybenzyl)-5-phenyl-1,2,4-triazole,
3-mercapto-4-(3,5-difluoro-4-hydroxybenzyl)-5-phenyl-1,2,4-triazole,
3-mercapto-4-benzyl-5-(4-t-butylphenyl)-1,2,4-triazole,
3-mercapto-4-(3,5-difluorobenzyl)-5-(4-t-butylphenyl)-1,2,4-triazole,
3-mercapto-4-benzyl-5-phenyl-1,2,4-trazole,
3-mercapto-4-phenyl-5-(4-t-butylphenyl)-1,2,4-triazole,
3-mercapto-4-(4-chlorophenyl)-5-(4-t-butylphenyl)-1,2,4-triazole,
3-mercapto-4-(4-bromophenyl)-5-(4-t-butylphenyl)-1,2,4-triazole,
3-mercapto-4-(4-fluorophenyl)-5-(4-t-butylphenyl)-1,2,4-triazole,
3-mercapto-4-(3-phenylpropyl)-5-(4-t-butylphenyl)-1,2,4-triazole,
3-mercapto-4-(3-phenylethyl)-5-(4-t-butylphenyl)-1,2,4-triazole,
3-mercapto-4-[3-(3,5-difluorophenyl)propyl]-5-(4-t-butylphenyl)-1,2,4-triazole,
3-mercapto-4-[3-(3,5-difluoro-4-methoxyphenyl)propyl]-5-(4-t-butylphenyl)-1,2,4-triazole,
3-mercapto-4-[3-(3,5-difluoro-4-hydroxyphenyl)propyl]-5-(4-t-butylphenyl)-1,2,4-triazole,
3-mercapto-4-benzyl-5 methyl-1,2,4-triazole,
3-mercapto-4-benzyl-5-n-propyl-1,2,4-triazole,
3-mercapto-4-benzyl-5-n-pentyl-1,2,4-triazole,
3-mercapto-4-benzyl-5-n-heptyl-1,2,4-triazole,
3-mercapto-4-benzyl-5-n-nonyl-1,2,4-triazole,
3-mercapto-4-benzyl-5-cyclohexyl-1,2,4-triazole,
3-mercapto-4-benzyl-5-t-butyl-1,2,4-triazole,
3-mercapto-4,5-dibenzyl-1,2,4-triazole,
3-mercapto-4-benzyl-5-phenethyl-1,2,4-triazole,
3-mercapto-4-benzyl-5-(4-methoxyphenyl)-1,2,4-triazole,
3-mercapto-4-benzyl-5-(3,4,5-trimethoxyphenyl)-1,2,4-triazole,
3-mercapto-4-benzyl-5-(4-chlorophenyl)-1,2,4-triazole,
3-mercapto-4-benzyl-5-(4-bromophenyl)-1,2,4-triazole, or
3-mercapto-4-benzyl-5-(3-bromophenyl)-1,2,4-triazole,
or a pharmaceutically acceptable salt thereof.

9. The use of a compound of formula (Ib) as defined in claim 7 in the manufacture of a medicament for inhibiting dopamine-β-hydroxylase activity.

10. A compound of formula (II)

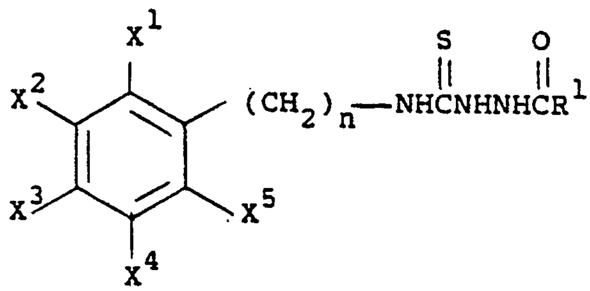

(II)

in which,
$X^1$ to $X^5$ are any accessible combination of hydrogen, halogen, $C_{1-6}$alkyl, $C_{1-6}$alkoxy, cyano, nitro, $SONH_2$, $SO_2NH_2$, $SO_2CH_3$, $SO_2CH_2F$, $SO_2CHF_2$, $SO_2CF_3$, $CF_3$, CHO, $CH_2OC_{1-6}$alkyl, or $CO_2C_{1-6}$alkyl;
and n and $R^1$ are as defined in claim 1.

11. A process for preparing a compound of formula (II) as defined in claim 10 which comprises reacting a compound of formula (III) with a compound of formula (IV) :

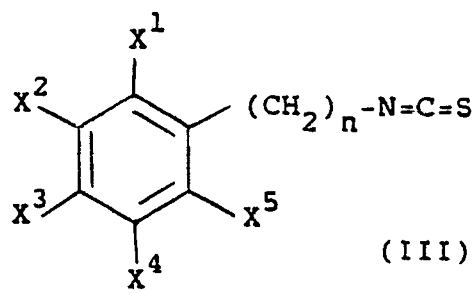

(III)

$R^1CONHNH_2$ (IV)
in which,
$X^1$ to $X^5$, n and $R^1$ are as defined in claim 10.

European Patent Office

# EUROPEAN SEARCH REPORT

Application number

EP 88312197.2

| Category | Citation of document with indication, where appropriate, of relevant passages | Relevant to claim | CLASSIFICATION OF THE APPLICATION (Int. Cl.4) |
|---|---|---|---|
| X | FR - A1 - 2 546 887 (UNI. PARIS). * Claim 1; table I * | 1-11 | C 07 D 249/12<br>C 07 C 159/00<br>A 61 K 31/41 |
| X | DE - A1 - 2 814 458 (CIBA-GEIGY) * Formula I; table I, example 12 * | 1-11 | |
| X | CHEMICAL ABSTRACTS, vol. 103, no. 3, July 22, 1985, Columbus, Ohio, USA<br>MALBEC, FREDERIQUE et al. "Derivatives of 2,4-dihydro-1,2,4-triazole-3-thione and 2-amino-1,3,4-thiadiazole from thiosemicarbazones of esters"<br>page 571, column 1, Abstract-no. 22 524w<br>& J. Heterocycl. Chem. 1984, 21(6), 1689-98 | 1-11 | **TECHNICAL FIELDS SEARCHED (Int. Cl.4)** |
| X | CHEMICAL ABSTRACTS, vol. 101, no. 25, December 17, 1984, Columbus, Ohio, USA<br>KHRIPAK, S.M. et al. "Acylation and iodination of 5-p-R-phenyl-1,2,4-triazole-3-thiones"<br>page 761, column 1, Abstract-no. 230 418a<br>& Khim. Geterotsikl. Soedin. 1984, (6), 843-6 | 1-11 | C 07 D 249/00<br>C 07 C 159/00 |

The present search report has been drawn up for all claims

| Place of search | Date of completion of the search | Examiner |
|---|---|---|
| VIENNA | 03-04-1989 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

-2-

EP 88312197.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 105, no. 9, September 1, 1986, Columbus, Ohio, USA<br>KNYSH, E.G. et al. "Synthesis and properties of 5-heteroarylthio-1,2,-4-triazoles"<br>page 17, column 1, Abstract-no. 72 087t<br>& Farm. Zh. (Kiev) 1986, (2), 49-51<br>-- | 1 | |
| X | CHEMICAL ABSTRACTS, vol. 99, no. 5, August 1, 1983, Colombus, Ohio, USA<br>KNISH, E.G. et al. "Synthesis, properties and biological activity of 5-(acylalkylthio)-1,2,4-triazoles"<br>page 512, column 2, Abstract-no. 38 421v<br>& Farm. Zh. (Kiev) 1983, (2), 64-5<br>-- | 1 | TECHNICAL FIELDS SEARCHED (Int. Cl.4) |
| P,A | US - A - 4 761 415 (FINKELSTEIN)<br>* Abstract *<br>-- | 1,9,10 | |
| A | US - A - 4 481 360 (GALL)<br>* Formula I *<br>-- | 1 | |
| A | US - A - 4 338 453 (GALL)<br>* Formula I *<br>-- | 1 | |

The present search report has been drawn up for all claims

| Place of search | Date of completion of the search | Examiner |
|---|---|---|
| VIENNA | 03-04-1989 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

Application number

-3-

EP 88312197.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | EP - A3 - 0 276 793 (MERRELL)<br>* Abstract *<br>-- | 1 | |
| A | EP - A1 - 0 189 774 (TOKUYAMA)<br>* Example 26 *<br>-- | 1 | |
| A | DE - A1 - 2 126 304 (VEB-BERLIN)<br>* Claim 1 *<br>-- | 12 | |
| A | GB - A - 2 030 565 (GULF OIL)<br>* Abstract *<br>---- | 12 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 03-04-1989 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document